# EUROPEAN PATENT APPLICATION

(11) **EP 2 614 879 A1**
(43) Date of publication of application: **17.07.2013**
(21) Application number: 11823470.7
(22) Date of filing: 01.09.2011
(51) Int. Cl.: B01D 61/36, B01D 61/58

(54) **DEHYDRATING DEVICE**

(30) Priority: 09.09.2010 JP 2010201860
(71) Applicant: Mitsubishi Heavy Industries, Ltd., Tokyo 108-8215 (JP)
(72) Inventor: TACHIBANA, Shinya, Tokyo 108-8215 (JP); TANAKA, Yukio, Tokyo 108-8215 (JP); OSORA, Hiroyuki, Tokyo 108-8215 (JP); HIRAYAMA, Haruaki, Tokyo 108-8215 (JP)
(74) Representative: Intès, Didier Gérard André
(86) International application number: PCT/JP2011/069878
(87) International publication number: WO 2012/032994

(57) **Abstract**

Provided is a dehydrator 1 configured to separate water from treated fluid, which includes a first tank 2 for storing the treated fluid before water is separated therefrom, a second tank 3 into which the treated fluid whose water has been separated therefrom flows, a separation membrane configured to separate water from the treated fluid, and a plurality of membrane container units 101 through 110 provided between the first tank 2 and the second tank 3 and in parallel to one another along a direction of flow of the treated fluid. The treated fluid is configured to reciprocate between the first tank 2 and the second tank 3 and is configured to pass through the plurality of membrane container units for a plurality of times.

## Description

### Technical Field

The present invention relates to a dehydrator for dehydrating a mixture (treated fluid) of water with ethanol which forms an azeotropic composition with water.

### Background Art

In recent years, ethanol has attracted attention as a fuel source alternative to petroleum fuels. However, for adopting ethanol as a fuel, it is necessary to purify, by distillation, a crude product obtained from a biomass such as corn, and then to perform dehydration until at least 99.5 wt% is reached.

Known conventional dehydration methods include a method for separating water from the treated fluid by the pervaporation method (penetrative vaporization (PV) method) using a water separation membrane (see, for example, Patent Literature 1). In the dehydrator discussed by Patent Literature 1, a plurality of water separation membranes is arranged so that the water separation membranes are arranged in series in the inside of a shell part. In this dehydrator, the treated fluid containing water passes through the serially arranged water separation membranes one after another. In this manner, water is separated from the treated fluid.

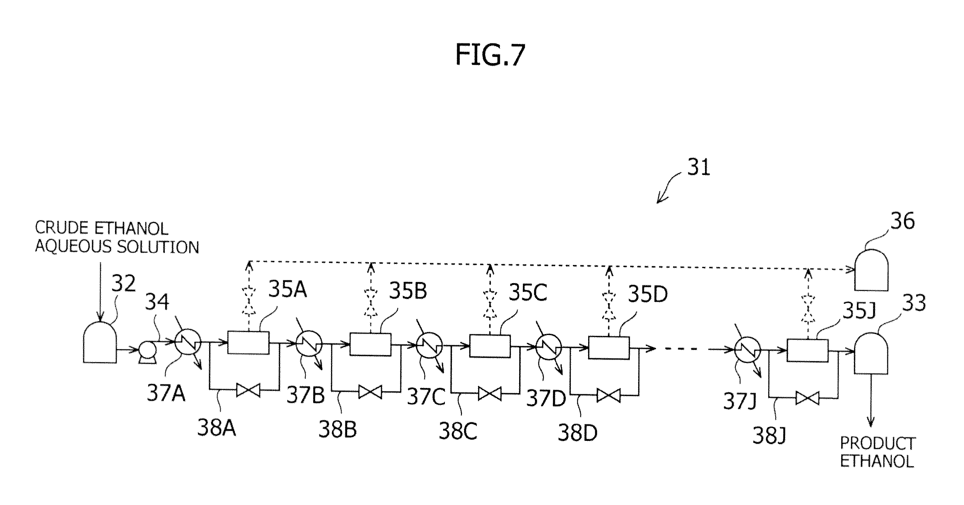

Fig. 7 is a schematic diagram which illustrates the entire conventional dehydrator. Referring to Fig. 7, a dehydrator 31 includes a first tank 32, to which the treated fluid (a crude ethanol aqueous solution) is supplied, a second tank 33, into which dehydrated product ethanol flows, a line 34 for allowing the treated fluid to flow from the first tank 2 to the second tank 3, a plurality of water separation membrane units 35A through 35J provided between the first tank 2 and the second tank 3, and a third tank 36, into which the water separated from the treated fluid flows.

As illustrated in Fig. 7, the plurality of water separation membrane units 35A through 35J is connected in series. Each of heat exchangers 37A through 37J is provided among the water separation membrane units 35A through 35J. In addition, for each of the plurality of water separation membrane units 35A through 35J, each of bypass lines 38A through 38J for bypassing the water separation membrane units 35A through 35J is provided.

### Citation List

### Patent Literature

PTL 1: Japanese Patent Application Laid-Open No. 2010-115596
PTL 2: US 2008/0,164,207 A1
PTL 3: WO 2008/111671 A1

### Summary of Invention

### Technical Problem

A first problem, which arises in the configuration illustrated in Fig. 7 and the conventional configuration discussed by Patent Literature 1, may arise because the water separation membrane units are arranged in series in this configuration. For example, if water separation membrane units are arranged in series and if any malfunction has occurred in a water separation membrane unit on an upstream side out of the water separation membrane units, the malfunction may affect the water separation membrane units on a downstream side. More specifically, if any malfunction has occurred in a water separation membrane unit on the upstream side, it becomes necessary to stop the entire dehydrator and to execute a maintenance operation. Accordingly, a problem may arise such that the dehydrator cannot be stably operated. In addition, in this case, although the operation of the dehydrator can be continued by using the bypass lines, the treated fluid may not be dehydrated until a predetermined concentration is achieved because the number of times the treated fluid passes through the water separation membrane units is reduced.

Furthermore, a second problem may arise such that in the configuration that uses water separation membranes as illustrated in Fig. 7 and as discussed by Patent Literature 1, the water separation performance may greatly vary according to the velocity of flow of the treated fluid.

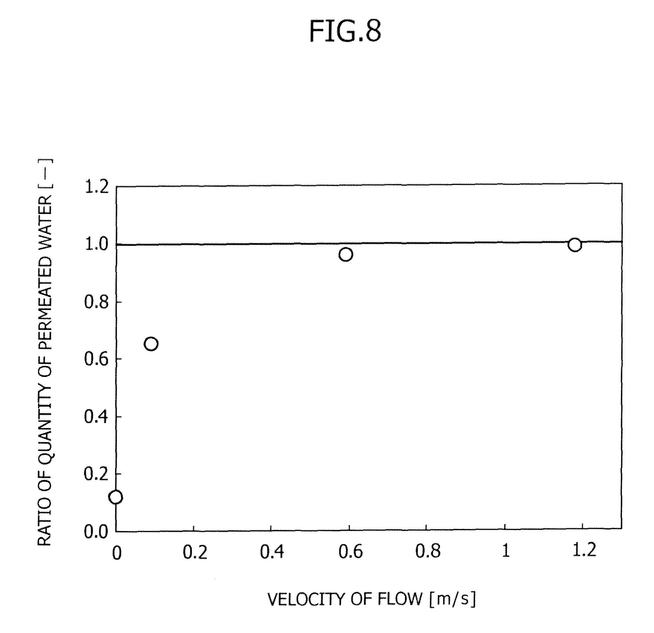

Fig. 8 illustrates a ratio of quantity of permeated water to the quantity of permeation of a matter (containing water and ethanol) that has permeated through water separation membranes. Referring to Fig. 8, the ratio of quantity of permeated water is about 1 at the velocity of flow of approximately 0.6 m/s. The ratio of quantity of permeated water to the quantity of matter permeated through the water separation membranes becomes lower as the velocity of flow of the treated fluid becomes lower. As described above, if water separation membranes are used and if the velocity of flow of the treated fluid is low, the ratio of permeated water to the matter permeated through the water separation membranes becomes low. As a result, the water separation performance may degrade.

In addition, if the velocity of flow of the treated fluid is low, the problem may arise such that concentration polarization may occur in the vicinity of the water separation membranes. The concentration polarization is a phenomenon that may occur in the vicinity of water separation membranes, in which a dissolved matter (water in the example illustrated in Fig. 8) that does not permeate through water separation membranes produce a gradient of concentration in the direction perpendicular to the water separation membrane.

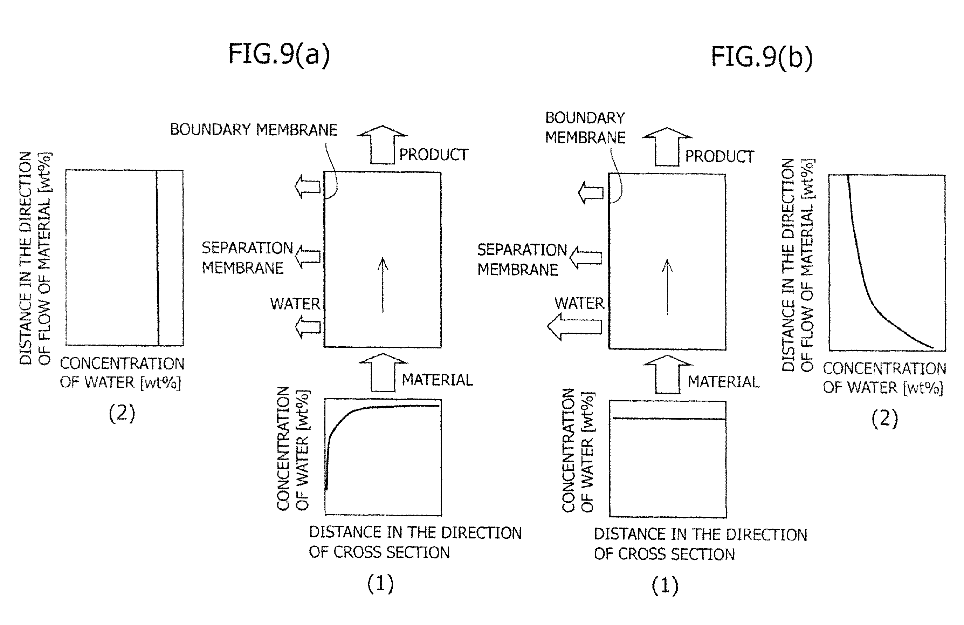

Figs. 9A and 9B illustrate a case where the concentration gradient is produced and another case where the concentration gradient is not produced. More specifically, Fig. 9A illustrates a case where the velocity of flow of the treated fluid is low. Fig. 9B illustrates a case where the velocity of flow of the treated fluid is high. As illustrated in a chart (1) of Fig. 9A, if the velocity of flow of the treated fluid is low, the concentration of water becomes lower as the distance to the water separation membrane becomes smaller while the concentration of water becomes higher as the distance to the water separation membrane in the direction perpendicular to the water separation membrane (in the direction of the cross section) becomes greater. As a result of such concentration polarization, referring to the change of the concentration of water in the direction of flow of the treated fluid illustrated in the chart (2) of Fig. 9A, the concentration of water hardly becomes low. Accordingly, it is known that the water separation performance is problematically low. On the other hand, as illustrated in the chart (1) of Fig. 9B, if the velocity of flow of the treated fluid is sufficiently high, water permeates through the water separation membranes. Accordingly, no concentration polarization occurs. As a result, referring to the change of the concentration of water in the direction of flow of the treated fluid illustrated in the chart (2) of Fig. 9B, the concentration of water gradually becomes lower. Accordingly, it is known that a sufficiently high water separation performance is achieved.

As described above, in the configuration that uses water separation membranes, a sufficiently high water separation performance cannot be achieved if the velocity of flow of the treated fluid becomes low. Accordingly, in this case, the treated fluid may not be dehydrated well enough to reach a predetermined concentration. Therefore, the configuration that uses water separation membranes requires certain measures for securely dehydrating the treated fluid well enough to reach a predetermined concentration.

The present invention is devised to solve the above-described problems. The object of the present invention is to provide a dehydrator which can be safely operated if any malfunction has occurred to a water separation membrane unit and which is capable of securely dehydrating the treated fluid well enough to reach the predetermined concentration.

### Solution to Problem

In order to solve the above-described problems arising in the conventional techniques, according to an aspect of the present invention, a dehydrator configured to separate water from treated fluid includes: a first tank configured to store the treated fluid before water is separated therefrom; a second tank into which the treated fluid whose water has been separated therefrom flows; and a plurality of membrane container units having a separation membrane configured to separate water from the treated fluid, and provided between the first tank and the second tank and in parallel to one another along a direction of flow of the treated fluid. The treated fluid is configured to reciprocate between the first tank and the second tank and is configured to pass through the plurality of membrane container units for a plurality of times.

According to another aspect of the present invention, the dehydrator further includes a first line configured to allow the treated fluid to reciprocate between the first tank and the second tank. In the dehydrator, the plurality of membrane container units is arranged on the first line in parallel to one another and the treated fluid is configured, after all the treated fluid has flowed from the first tank to the second tank via the plurality of membrane container units provided on the first line and no treated fluid remains in the first tank, to flow from the second tank to the first tank via the plurality of membrane container units provided on the first line.

According to yet another aspect of the present invention, the dehydrator further includes the first line configured to allow the treated fluid to flow from the first tank to the second tank, and a second line configured to allow the treated fluid to flow from the second tank to the first tank. In the dehydrator, the plurality of membrane container units is arranged in parallel to one another and the treated fluid is configured, after all the treated fluid has flowed from the first tank to the second tank via the plurality of membrane container units provided on the first line and no treated fluid remains in the first tank, to flow from the second tank to the first tank via the second line.

According to still yet another aspect of the present invention, the dehydrator further includes the first line configured to allow the treated fluid to flow from the first tank to the second tank, and the second line configured to allow the treated fluid to flow from the second tank to the first tank. In the dehydrator, the plurality of membrane container units is divided into a first unit group including units provided on the first line and in parallel to one another and a second unit group including units provided on the second line and in parallel to one another, and the treated fluid is configured, after all the treated fluid has flowed from the first tank to the second tank via the first unit group provided on the first line and no treated fluid remains in the first tank, to flow from the second tank to the first tank via the second unit group provided on the second line.

According to still yet another aspect of the present invention, the dehydrator further includes the first line configured to allow the treated fluid to flow from the first tank to the second tank, the second line configured to allow the treated fluid to flow from the second tank to the first tank, and at least one intermediate tank provided between the first tank and the second tank and connected to both the first line and the second line. In the dehydrator, the first line is divided into a plurality of first fluid lines provided across the intermediate tank and the second line is divided into a plurality of second fluid lines provided across the intermediate tank, the plurality of membrane container units is divided into unit groups including as many units as a total number of the first fluid line and the second fluid line and units included in the unit group of the plurality of membrane container units are provided in parallel to one another on each of the first fluid line and the second fluid line, the treated fluid is configured to flow from the first tank to the second tank successively via the units included in the unit group provided on the first line and the intermediate tank and is configured to flow from the second tank to the first tank successively via the units included in the unit group provided on the second line and the intermediate tank, the second line includes at least one bypass line, and the bypass line is configured to bypass one or more units of the unit group provided on the second line, to allow the treated fluid to return to the intermediate tank or the first tank.

### Advantageous Effects of Invention

According to the dehydrator of an aspect of the present invention, because a plurality of membrane container units is provided in parallel to one another, if any malfunction has occurred to any water separation membrane unit, the maintenance and the replacement can be carried out only for the water separation membrane unit to which the malfunction has occurred without stopping the entire dehydrator. Accordingly, it is enabled to stably operate the dehydrator.

In addition, although the velocity of flow of the treated fluid may become low and the performance of water separation by the membrane container units may become low if a plurality of membrane container units is provided in parallel to one another, the treated fluid can be securely dehydrated well enough to reach a predetermined concentration by allowing the treated fluid to reciprocate between the first tank and the second tank and by allowing the treated fluid to repeatedly pass through the membrane container units.

Furthermore, according to the dehydrator of the present invention, the second line includes at least one bypass line and the bypass line is configured to bypass one or more units included in the unit group provided on the second line to allow the treated fluid to return to the intermediate tank or the first tank. Accordingly, the number of times the treated fluid is to pass through the membrane container units can be appropriately changed. As a result, the concentration of the treated fluid can be finely adjusted.

### Brief Description of Drawings

Fig. 1 is a schematic diagram illustrating the entire dehydrator according to a first exemplary embodiment of the present invention.
Fig. 2 is a diagram illustrating a trial calculation of the transition of a concentration of ethanol obtained by dehydrating a crude ethanol aqueous solution by using the dehydrator according to the first exemplary embodiment of the present invention.
Fig. 3 is a schematic diagram illustrating the entire dehydrator according to a second exemplary embodiment of the present invention.
Fig. 4 is a schematic diagram illustrating the entire dehydrator according to a third exemplary embodiment of the present invention.
Fig. 5 is a schematic diagram illustrating the entire dehydrator according to a fourth exemplary embodiment of the present invention.
Fig. 6 is a schematic diagram illustrating the entire dehydrator according to a fifth exemplary embodiment of the present invention.
Fig. 7 is a schematic diagram illustrating the entire conventional dehydrator.
Fig. 8 is a diagram illustrating a ratio of the quantity of permeation of water to the quantity of permeation of a matter (containing water and ethanol) that has permeated through water separation membranes.
Fig. 9A is a diagram illustrating a case in which concentration polarization has occurred because the velocity of flow of treated fluid is low.
Fig. 9B is a diagram illustrating a case in which the concentration polarization has not occurred because the velocity of flow of the treated fluid is high.

### Description of Embodiments

### First Exemplary Embodiment

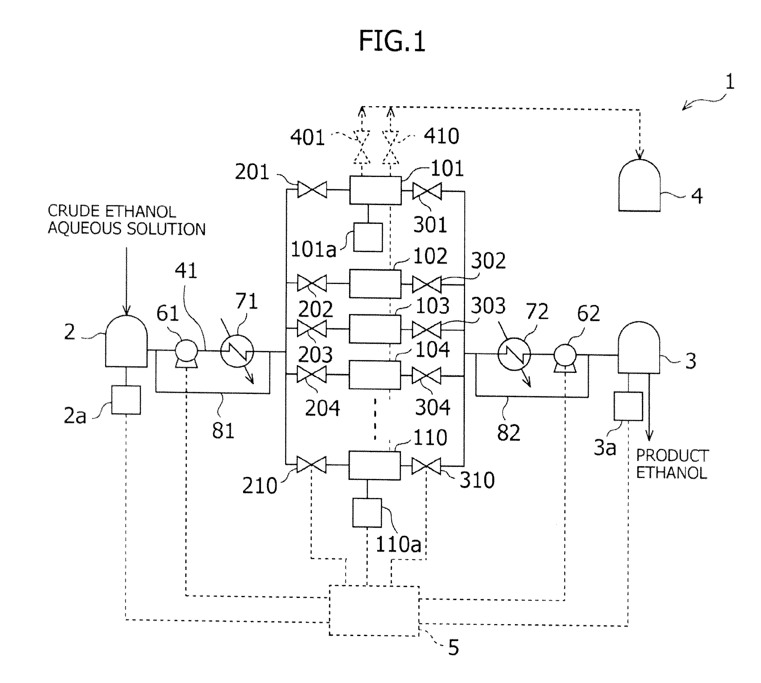

Hereinbelow, a dehydrator according to the first exemplary embodiment of the present invention will be described below with reference to the attached drawings. Fig. 1 is a schematic diagram illustrating the entire dehydrator according to the present exemplary embodiment.

The dehydrator 1 according to the present exemplary embodiment is configured to dehydrate a crude ethanol aqueous solution used as treated fluid. The inventors assume approximately 91 wt % of the concentration of ethanol for the concentration of the crude ethanol aqueous solution. The dehydrator 1 is configured to treat and purify crude ethanol containing ethanol as an organic component to finally produce product ethanol (absolute ethanol) whose ethanol concentration is in the range of 99.5 to 99.8 wt %.

Referring to Fig. 1, the dehydrator 1 includes a first tank 2, to which treated fluid (a crude ethanol aqueous solution) is supplied, a second tank 3, into which the dehydrated product ethanol flows, a first line 41 for allowing the treated fluid to reciprocate between the first tank 2 and the second tank 3, a plurality of (ten in the present exemplary embodiment) water separation membrane units 101 through 110 provided to the first line 41, a third tank 4, into which water separated from the treated fluid flows, and a control unit 5 configured to control the operation of the entire dehydrator 1.

As illustrated in Fig. 1, the first tank 2 is provided with a first ethanol concentration meter 2a. In addition, the second tank 3 is provided with a second ethanol concentration meter 3a. The first ethanol concentration meter 2a and the second ethanol concentration meter 3a are connected to the control unit 5. Detection signals are transmitted from the first ethanol concentration meter 2a and the second ethanol concentration meter 3a to the control unit 5.

As illustrated in Fig. 1, the first line 41 is provided with a first pump 61, which is provided at a location close to the first tank 2, and a second pump 62, which is provided at a location close to the second tank 3. The first pump 61 and the second pump 62 are connected to the control unit 5. The first pump 61 is configured to pump the treated fluid stored in the first tank 2 into the second tank 3 under control of the control unit 5. The second pump 62 also is configured to pump the treated fluid stored in the second tank 3 into the first tank 2 under control of the control unit 5. With the above-described configuration, the treated fluid is allowed to reciprocate through the first line 41 between the first tank 2 and the second tank 3.

As illustrated in Fig. 1, a plurality of water separation membrane units 101 through 110 is provided on the first line 41 in parallel to one another. First open-close valves 201 through 210 are provided on the side of the first tank 2 of the water separation membrane units 101 through 110. In addition, second open-close valves 301 through 310 are provided on the side of the second tank 3 of the water separation membrane units 101 through 110. As illustrated in Fig. 1, the first open-close valves 201 through 210 and the second open-close valves 301 through 310 are connected to the control unit 5 and are configured to open and close under control of the control unit 5.

Each of the water separation membrane units 101 through 110 is provided with a membrane container (not illustrated), which will be described below, and is configured to separate water by the pervaporation method in which the supply side is a liquid phase and the permeation side is a gas phase. In the present exemplary embodiment, for the membrane container of the water separation membrane units 101 through 110, a flow path for the treated fluid is referred to as the "supply side" and the outside of the membrane container is referred to as the "permeation side".

In the present exemplary embodiment, the membrane container is constituted by a combination of a plurality of tubular-type water separation membranes or a monolith-type water separation membrane and has a columnar shape. As the membrane container, an inorganic porous membrane in which holes on the order of nanometers or smaller are controlled precisely can be used. The porous membrane having fine holes achieves a molecule sieving effect of allowing small-molecule gases to pass through and exclude large-molecule gases, and exhibits a behavior of activation diffusion in which the permeation factor thereof increases with the increase in temperature. As an example of a porous membrane having fine holes, a carbon membrane, a silica membrane, and a zeolite membrane can be mentioned.

Also, the inorganic water separating membrane described in Japanese Patent No. 2808479 can also be applied. The inorganic water separating membrane described in Japanese Patent No. 2808479 is an acid-resistant composite separation membrane obtained by carrying silica gel obtained through hydrolysis of alkoxysilane containing an ethoxy group or methoxy group in the fine holes of an inorganic porous body. Note that, as a porous base member on which the inorganic water separation membrane is supported, a base member of a ceramic such as alumina, silica, zirconia, or titania is usually used, and a preferable base member is a tubular base member having multiple flow paths (inner tubes) which extend in the longitudinal direction and each of which have a circular cross-section. The inorganic water separation membrane is formed in a way to cover inner walls of such inner tubes.

Note that, besides the inorganic water separation membrane, an organic membrane such as a polyvinyl alcohol membrane, a polyimide membrane or a polyamide membrane can be used as the water separation membrane. Note that the material and the size of the membrane container and the diameter, the quantity, and the like of the flow paths can be selected by a person having ordinary skill in the art according to their purpose of use.

The dehydration treatment by the membrane container is carried out by using a decompression device (not illustrated). By decompressing the permeation side of the membrane container by using the decompression device, the water contained in the treated fluid that passes through vacuum device the flow path of the membrane container becomes steam. The steam is drawn out to the permeation side. As illustrated in Fig. 1, the water separation membrane units 101 through 110 are respectively connected to the third tank 4 via the steam valves 401 through 410. The steam drawn out by each of the water separation membrane units 101 through 110 flows into the third tank 4.

As illustrated in Fig. 1, on the first line 41, a first heat exchanger 71 is provided between the first pump 61 and the plurality of water separation membrane units 101 through 110. In addition, on the first line 41, a second heat exchanger 72 is provided between the second pump 62 and the plurality of water separation membrane units 101 through 110. The first heat exchanger 71 and the second heat exchanger 72 apply heat to raise the temperature of the treated fluid that flows into the water separation membrane units 101 through 110 to prevent the temperature of the treated fluid from dropping.

As illustrated in Fig. 1, on the first line 41, a first bypass tube 81 for bypassing the first pump 61 and the first heat exchanger 71 is provided. Both ends of the first bypass tube 81 are connected to the first line 41 via a branch valve (not illustrated). In addition, on the first line 41, a second bypass tube 82 for bypassing the second pump 62 and the second heat exchanger 72 is provided. Both ends of the second bypass tube 82 are connected to the first line 41 via a branch valve (not illustrated). Note that the branch valve is connected to the control unit 5 and is configured to open and close under control of the control unit 5.

As illustrated in Fig. 1, the water separation membrane units 101 through 110 are provided with detectors 101a through 110a configured to detect malfunction occurring on the membrane container, respectively. The detectors 101a through 110a are configured to continuously monitor the state of the membrane containers and can carry out on-line detection of malfunction. For example, if the quantity of permeation is decreased by clogging, the detectors 101a through 110a detect that the decrease in outlet temperature on the supply side is small. Also, if the quantity of permeation is increased by a malfunction occurring in the membrane container, the detectors 101a through 110a can detect that the decrease in temperature on the permeation side is large. Furthermore, the detectors 101a through 110a can detect that the outlet concentration on the supply side has been changed due to the change in the quantity of permeation.

As illustrated in Fig. 1, the detectors 101a through 110a are connected to the control unit 5 and are configured to transmit detection signals to the control unit 5. When the control unit 5 receives the detection signal which indicates that malfunction has occurred to the membrane container, the control unit 5 executes control for closing both the first open-close valves 201 through 210 and the second open-close valves 301 through 310 corresponding to the water separation membrane unit to which the malfunction has occurred, among the water separation membrane units 101 through 110. With the above-described configuration, the maintenance and the replacement can be carried out only for the water separation membrane unit to which the malfunction has occurred, among the water separation membrane units 101 through 110.

Now, an operation of the dehydrator 1 according to the present exemplary embodiment will be described below with reference to the attached drawings.

As illustrated in Fig. 1, at the start of the operation, the treated fluid (a crude ethanol aqueous solution) is supplied to the first tank 2. The treated fluid is intermittently supplied and the supply of the treated fluid is stopped when the first tank 2 is filled with the treated fluid. Then, the control unit 5 controls the branch valve provided between the first line 41 and the first bypass tube 81 to allow the treated fluid to flow into the first line 41. After that, the first pump 61 pumps the treated fluid from the first tank 2 to the second tank 3. The treated fluid that flows through the first line 41 is heated by the first heat exchanger 71 and is allowed to flow into the plurality of water separation membrane units 101 through 110 provided in parallel to one another. The steam drawn out by each of the water separation membrane units 101 through 110 during the operation described above flows into the third tank 4. Then, the control unit 5 controls the branch valve provided between the first line 41 and the second bypass tube 82 to allow the treated fluid to flow into the second bypass tube 82. Then, the treated fluid that has passed through the second bypass tube 82 flows into the second tank 3.

After all the treated fluid has flowed from the first tank 2 to the second tank 3, the control unit 5 receives a concentration measured by the second ethanol concentration meter 3a of the second tank 3. If the concentration measured by the second ethanol concentration meter 3a has not reached a predetermined concentration yet, then the control unit 5 executes control for allowing the treated fluid to flow from the second tank 3 to the first tank 2 in the following manner. At the start of the control, the control unit 5 controls the branch valve provided between the first line 41 and the second bypass line 82 to allow the treated fluid to flow into the first line 41. Consequently, the second pump 62 pumps the treated fluid from the second tank 3 to the first tank 2. The treated fluid that flows through the first line 41 is heated by the second heat exchanger 72 and is allowed to flow into the plurality of water separation membrane units 101 through 110 provided in parallel to one another. The steam drawn out by each of the water separation membrane units 101 through 110 during the above-described operation flows into the third tank 4. Then, the control unit 5 controls the branch valve provided between the first line 41 and the first bypass tube 81 to allow the treated fluid to flow into the first bypass tube 81. Then, the treated fluid that has passed through the first bypass tube 81 flows into the first tank 2. Then, after all the treated fluid has flowed from the second tank 3 to the first tank 2, the control unit 5 receives a concentration measured by the first ethanol concentration meter 2a of the first tank 2. Then, the control unit 5 detects whether the concentration measured by the first ethanol concentration meter 2a has reached the predetermined concentration. If it is detected that the concentration has not reached the predetermined concentration yet, then the control unit 5 executes control for allowing the treated fluid to flow from the first tank 2 into the second tank 3 again. The control unit 5 executes the operation described above until the concentration of the treated fluid reaches the predetermined concentration. Then, the treated fluid whose concentration has reached the predetermined concentration is finally recovered from the second tank 3 as product ethanol.

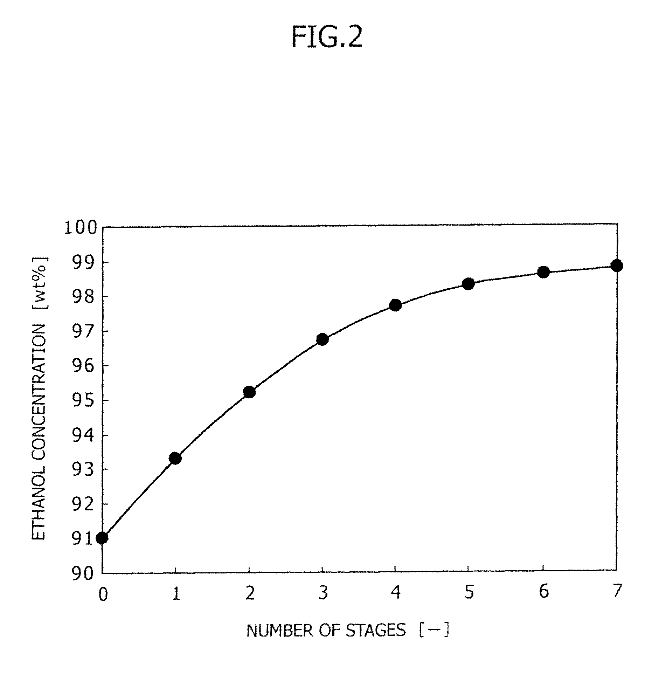

Fig. 2 is a diagram illustrating a trial calculation of the transition of a concentration of ethanol obtained by dehydrating a crude ethanol aqueous solution by using the dehydrator 1 according to the present exemplary embodiment. The "number of stages" taken on the horizontal axis of the example illustrated in Fig. 2 corresponds to the number of times the treated fluid has passed through the plurality of water separation membrane units 101 through 110. For example, in the present exemplary embodiment, the number of stages of 1 refers to the timing at which the treated fluid has flowed from the first tank 2 to the second tank 3 once and the number of stages of 2 refers to the timing at which the treated fluid has flowed back from the second tank 3 and again into the first tank 2. As illustrated in Fig. 2, according to the dehydrator 1 of the present exemplary embodiment, it can be known that the ethanol concentration is increased every time the treated fluid passes through the plurality of water separation membrane units 101 through 110 and the ethanol concentration approaches the predetermined concentration of the product ethanol.

According to the dehydrator 1 of the present exemplary embodiment, the plurality of water separation membrane units 101 through 110 is provided on the first line 41 in parallel to one another. Accordingly, if any malfunction has occurred to any of the water separation membrane units 101 through 110, the maintenance and the replacement can be carried out only for the water separation membrane unit to which the malfunction has occurred, among the water separation membrane units 101 through 110, without stopping the entire dehydrator 1. Accordingly, it is enabled to stably operate the dehydrator 1.

In addition, for example, in the configuration in which the plurality of water separation membrane units 101 through 110 is arranged in parallel to one another, if the serially arranged pumps used in the conventional configuration are used as they are with the serial arrangement, the velocity of flow of the treated fluid may decrease. As a result, the performance of water separation by the water separation membrane units 101 through 110 may degrade. In addition, even if the velocity of flow of the treated fluid is increased by increasing the capacity of the first pump 61 and the second pump 62 in the configuration in which the water separation membrane units 101 through 110 are arranged in parallel to one another, the treated fluid may not be securely dehydrated well enough to reach the predetermined concentration because the contact time of the treated fluid with the membrane container becomes shorter compared to the conventional configuration in which the water separation membrane units 101 through 110 are arranged in series. The dehydrator 1 according to the present exemplary embodiment is configured to solve the above-described problem. Furthermore, the dehydrator 1 is configured to allow the treated fluid to reciprocate between the first tank 2 and the second tank 3 and to repeatedly pass through the water separation membrane units 101 through 110. With the above-described configuration, the dehydrator 1 can securely dehydrate the treated fluid well enough to reach the predetermined concentration.

### Second Exemplary Embodiment

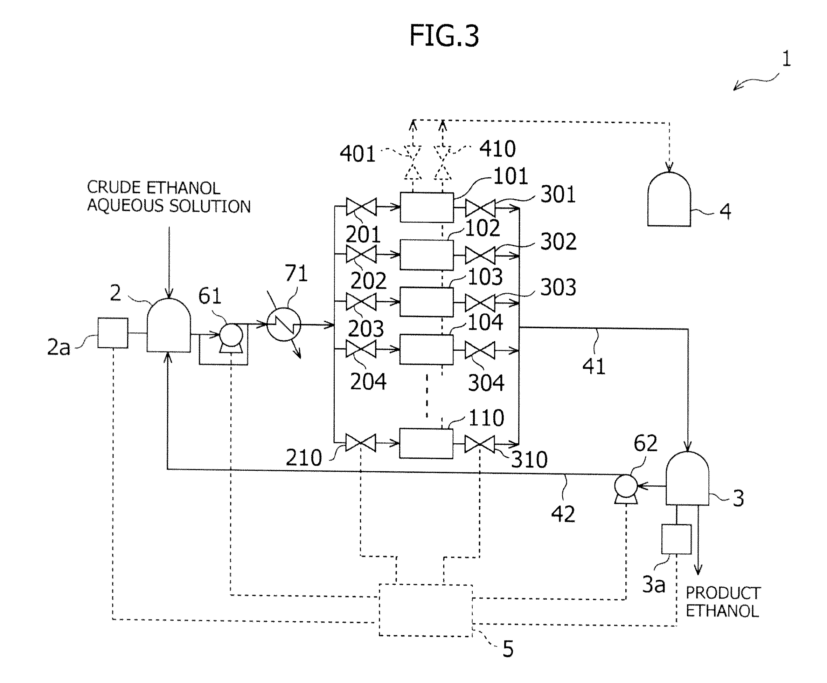

Now, the dehydrator 1 according to a second exemplary embodiment of the present invention will be described below with reference to the attached drawings. Fig. 3 is a schematic diagram illustrating the entire dehydrator according to the present exemplary embodiment. Note that the components similar to those of the above-described exemplary embodiment are provided with the same reference signs. Accordingly, the detailed description thereof will not be repeated here.

In the present exemplary embodiment, as illustrated in Fig. 3, the dehydrator 1 includes the first line 41 for allowing the treated fluid to flow from the first tank 2 to the second tank 3 and a second line 42 for allowing the treated fluid to flow from the second tank 3 to the first tank 2.

As illustrated in Fig. 3, the first line 41 includes the first pump 61 provided at a location close to the first tank 2 and the second line 42 includes the second pump 62 provided at a location close to the second tank 3. The first pump 61 and the second pump 62 are connected to the control unit 5. The first pump 61 is configured to pump the treated fluid from the first tank 2 to the second tank 3 under control of the control unit 5. The second pump 62 also is configured to pump the treated fluid from the second tank 3 into the first tank 2 under control of the control unit 5.

As illustrated in Fig. 3, the plurality of water separation membrane units 101 through 110 is provided on the first line 41 in parallel to one another. In addition, on the first line 41, a first heat exchanger 71 is provided between the first pump 61 and the plurality of water separation membrane units 101 through 110. On the other hand, in the present exemplary embodiment, no water separation membrane unit or heat exchanger is provided for the second line 42. To paraphrase this, the second line 42 is a dedicated line for returning the treated fluid from the second tank 3 to the first tank 2.

Note that although not illustrated in Fig. 3, similarly to the first exemplary embodiment described above, each of the water separation membrane units 101 through 110 includes a detector for detecting malfunction that may occur on the membrane container. The detector is connected to the control unit 5 and a detection signal is transmitted from the detector to the control unit 5. When the control unit 5 receives the detection signal which indicates that malfunction has occurred to the membrane container, the control unit 5 executes control for closing both the first open-close valves 201 through 210 and the second open-close valves 301 through 310 corresponding to the water separation membrane unit to which the malfunction has occurred, among the water separation membrane units 101 through 110. With the above-described configuration, the maintenance and the replacement can be carried out only for the water separation membrane unit to which the malfunction has occurred, among the water separation membrane units 101 through 110.

Now, an operation of the dehydrator 1 according to the present exemplary embodiment will be described below with reference to the attached drawings.

As illustrated in Fig. 3, at the start of the operation, the treated fluid (a crude ethanol aqueous solution) is supplied to the first tank 2. The treated fluid is intermittently supplied and the supply of the treated fluid is stopped when the first tank 2 is filled with the treated fluid. After that, the first pump 61 pumps the treated fluid from the first tank 2 to the second tank 3. The treated fluid that flows through the first line 41 is heated by the first heat exchanger 71 and is allowed to flow into the plurality of water separation membrane units 101 through 110 provided in parallel to one another. The steam drawn out by each of the water separation membrane units 101 through 110 during the operation described above flows into the third tank 4. Then, after passing through the plurality of water separation membrane units 101 through 110, the treated fluid flows into the second tank 3.

After all the treated fluid has flowed from the first tank 2 to the second tank 3, the control unit 5 receives a concentration measured by the second ethanol concentration meter 3a of the second tank 3. If the concentration measured by the second ethanol concentration meter 3a has not reached a predetermined concentration yet, then the control unit 5 controls the second pump 62. By executing the above-described operation, the treated fluid flows from the second tank 3 to the first tank 2 via the second line 42. Then, after all the treated fluid has flowed from the second tank 3 to the first tank 2, the control unit 5 executes control for allowing the treated fluid to flow from the first tank 2 to the second tank 3 again. The control unit 5 executes the operation described above until the concentration of the treated fluid reaches the predetermined concentration. Then, the treated fluid whose concentration has reached the predetermined concentration is finally recovered from the second tank 3 as product ethanol.

According to the dehydrator 1 of the present exemplary embodiment, the plurality of water separation membrane units 101 through 110 is provided on the first line 41 in parallel to one another. Accordingly, if any malfunction has occurred to any of the water separation membrane units 101 through 110, the maintenance and the replacement can be carried out only for the water separation membrane unit to which the malfunction has occurred, among the water separation membrane units 101 through 110, without stopping the entire dehydrator 1. Accordingly, it is enabled to stably operate the dehydrator 1.

In addition, according to the dehydrator 1 of the present exemplary embodiment, because the dehydrator 1 includes the second line 42 for allowing the treated fluid to return from the second tank 3 to the first tank 2, the treated fluid can be securely dehydrated well enough to reach the predetermined concentration by allowing the treated fluid to repeatedly pass through the water separation membrane units 101 through 110. Furthermore, according to the dehydrator 1 of the present exemplary embodiment, because the treated fluid flows in one direction by providing two lines including the first line 41 and the second line 42, the configuration of the dehydrator 1 can be more simplified. In addition, because the treated fluid is allowed to flow from the second tank 3 to the first tank 2 by using the second line 42 before all the treated fluid completely flows from the first tank 2, the capacity of the second tank 3 can become smaller than that in the configuration in which only one line for allowing the treated fluid to reciprocate therethrough is provided.

### Third Exemplary Embodiment

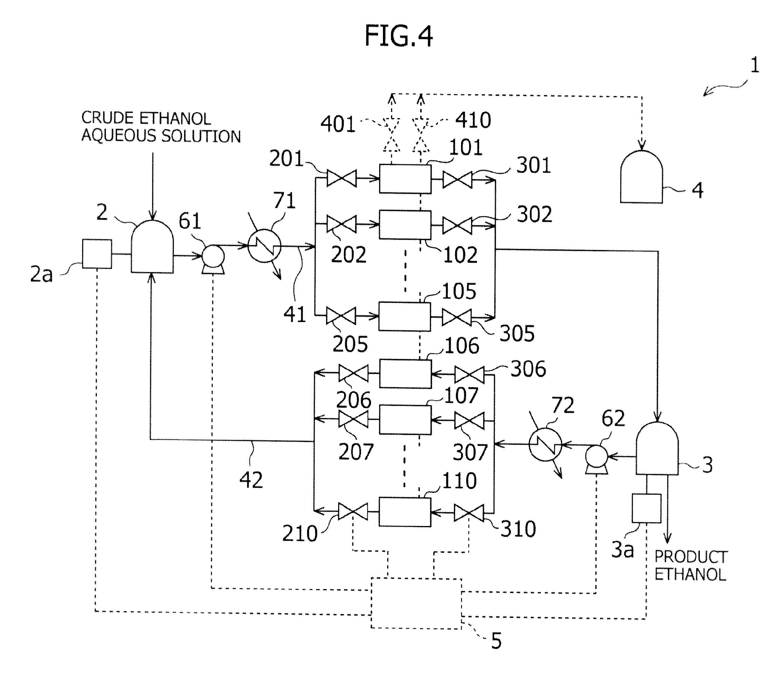

Now, the dehydrator 1 according to a third exemplary embodiment of the present invention will be described below with reference to the attached drawings. Fig. 4 is a schematic diagram illustrating the entire dehydrator 1 according to the present exemplary embodiment. Note that the components similar to those of the above-described exemplary embodiments are provided with the same reference signs. Accordingly, the detailed description thereof will not be repeated here.

In the present exemplary embodiment, as illustrated in Fig. 4, the dehydrator 1 includes the first line 41 for allowing the treated fluid to flow from the first tank 2 to the second tank 3 and the second line 42 for allowing the treated fluid to flow from the second tank 3 to the first tank 2.

As illustrated in Fig. 4, the first line 41 includes the first pump 61 provided at a location close to the first tank 2 and the second line 42 includes the second pump 62 provided at a location close to the second tank 3. The first pump 61 and the second pump 62 are connected to the control unit 5. The first pump 61 is configured to pump the treated fluid from the first tank 2 to the second tank 3 under control of the control unit 5. The second pump 62 also is configured to pump the treated fluid contained in the second tank 3 into the first tank 2 under control of the control unit 5.

As illustrated in Fig. 4, in the present exemplary embodiment, the plurality of water separation membrane units 101 through 110 is divided into a first unit group including the units 101 through 105 and a second unit group including the units 106 through 110. In addition, the units 101 through 105 of the first unit group are provided on the first line 41 in parallel to one another and the units 106 through 110 of the second unit group are provided on the second line 42 in parallel to one another.

As illustrated in Fig. 4, on the first line 41, the first heat exchanger 71 is provided between the first pump 61 and the first unit group including the units 101 through 105. On the other hand, on the second line 42, the heat exchanger 72 is provided between the second pump 62 and the second unit group including the units 106 through 110.

Note that although not illustrated in Fig. 4, similarly to the first exemplary embodiment described above, each of the water separation membrane units 101 through 110 includes a detector for detecting the malfunction occurring on the membrane container. The detector is connected to the control unit 5 and a detection signal is transmitted from the detector to the control unit 5. When the control unit 5 receives the detection signal which indicates that malfunction has occurred to the membrane container, the control unit 5 executes control for closing both the first open-close valves 201 through 210 and the second open-close valves 301 through 310 corresponding to the water separation membrane unit to which the malfunction has occurred, among the water separation membrane units 101 through 110. With the above-described configuration, the maintenance and the replacement can be carried out only for the water separation membrane unit to which the malfunction has occurred, among the water separation membrane units 101 through 110.

Now, an operation of the dehydrator 1 according to the present exemplary embodiment will be described below with reference to the attached drawings.

As illustrated in Fig. 4, at the start of the operation, the treated fluid (a crude ethanol aqueous solution) is supplied to the first tank 2. The treated fluid is intermittently supplied and the supply of the treated fluid is stopped when the first tank 2 is filled with the treated fluid. After that, the first pump 61 pumps the treated fluid from the first tank 2 to the second tank 3. The treated fluid that flows through the first line 41 is heated by the first heat exchanger 71 and is allowed to flow into the first unit group including the units 101 through 105 arranged in parallel to one another. The steam drawn out by the first unit group including the units 101 through 105 during the operation described above flows into the third tank 4. Then, after passing through the first unit group including the units 101 through 105, the treated fluid flows into the second tank 3.

After all the treated fluid has flowed from the first tank 2 to the second tank 3, the control unit 5 receives a concentration measured by the second ethanol concentration meter 3a of the second tank 3. If the concentration measured by the second ethanol concentration meter 3a has not reached a predetermined concentration yet, then the control unit 5 controls the second pump 62. By executing the above-described operation, the treated fluid flows from the second tank 3 to the first tank 2 via the second line 42. The treated fluid that flows through the second line 42 is heated by the second heat exchanger 72 and is then allowed to flow into the second unit group including the units 106 through 110 arranged in parallel to one another. The steam drawn out by the second unit group including the units 106 through 110 during the operation described above flows into the third tank 4. Then, after passing through the second unit group including the units 106 through 110, the treated fluid flows into the first tank 2. The above-described operation is repeated until the concentration of the treated fluid reaches the predetermined concentration. Then, the treated fluid whose concentration has reached the predetermined concentration is finally recovered from the second tank 3 as product ethanol.

According to the dehydrator 1 of the present exemplary embodiment, the plurality of water separation membrane units 101 through 110 is divided into the first unit group including the units 101 through 105 and the second unit group including the units 106 through 110 and the units 101 through 105 of the first unit group are provided on the first line 41 in parallel to one another and the units 106 through 110 of the second unit group are provided on the second line 42 in parallel to one another. Accordingly, if any malfunction has occurred to any of the water separation membrane units 101 through 110, the maintenance and the replacement can be carried out only for the water separation membrane unit to which the malfunction has occurred, among the water separation membrane units 101 through 110, without stopping the entire dehydrator 1. Accordingly, it is enabled to stably operate the dehydrator 1.

In addition, according to the dehydrator 1 of the present exemplary embodiment, because the second line 42 for allowing the treated fluid to return from the second tank 3 to the first tank 2 is provided, the treated fluid can be securely dehydrated well enough to reach the predetermined concentration by allowing the treated fluid to repeatedly pass through the water separation membrane units 101 through 110. In addition, according to the dehydrator 1 of the present exemplary embodiment, the plurality of water separation membrane units 101 through 110 is divided into two unit groups. Accordingly, the decrease of the velocity of flow of the treated fluid can be suppressed. As a result, the decrease of the performance of water separation by the membrane container can be suppressed.

### Fourth Exemplary Embodiment

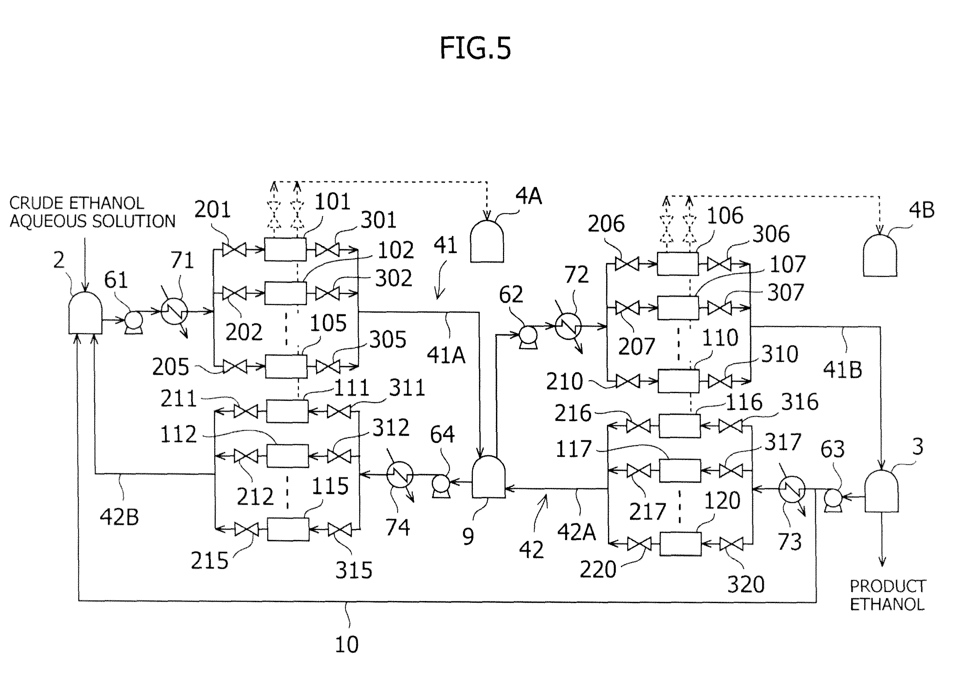

Now, the dehydrator 1 according to a fourth exemplary embodiment of the present invention will be described below with reference to the attached drawings. Fig. 5 is a schematic diagram illustrating the entire dehydrator 1 according to the present exemplary embodiment. Note that the components similar to those of the above-described exemplary embodiments are provided with the same reference signs. Accordingly, the detailed description thereof will not be repeated here.

In the present exemplary embodiment, as illustrated in Fig. 5, the dehydrator 1 includes an intermediate tank 9, which is provided between the first tank 2 and the second tank 3. The intermediate tank 9 is connected to both the first line 41 for allowing the treated fluid to flow from the first tank 2 to the second tank 3 and the second line 42 for allowing the treated fluid to flow from the second tank 3 to the first tank 2. As illustrated in Fig. 5, the first line 41 includes an upstream side fluid line 41A for allowing the treated fluid to flow from the first tank 2 to the intermediate tank 9 and a downstream side fluid line 41B for allowing the treated fluid to flow from the intermediate tank 9 to the second tank 3. In addition, the second line 42 is constituted by an upstream side fluid line 42A for allowing the treated fluid to flow from the second tank 3 in the intermediate tank 9 and a downstream side fluid line 42B for allowing the treated fluid to flow from the intermediate tank 9 to the first tank 2. Note that although one intermediate tank 9 is provided in the example illustrated in Fig. 5, two or more intermediate tanks 9 can be provided. If two or more intermediate tanks are provided, water separation membrane units can be provided in parallel to one another among the respective intermediate tanks.

As illustrated in Fig. 5, the upstream side fluid line 41A of the first line 41 includes the first pump 61, which is provided at a location close to the first tank 2, and the downstream side fluid line 41B of the first line 41 includes the second pump 62, which is provided at a location close to the intermediate tank 9. Furthermore, the upstream side fluid line 42A of the second line 42 includes a third pump 63, which is provided at a location close to the second tank 3, and the downstream side fluid line 42B of the second line 42 includes a fourth pump 64, which is provided at a location close to the intermediate tank 9.

In the present exemplary embodiment, as illustrated in Fig. 5, the dehydrator 1 includes twenty water separation membrane units 101 through 120. The plurality of water separation membrane units 101 through 120 is divided into the first unit group including the units 101 through 105, the second unit group including the units 106 through 110, a third unit group including the units 111 through 115, and a fourth unit group including the units 116 through 120.

As illustrated in Fig. 5, the units 101 through 105 of the first unit group are provided on the upstream side fluid line 41A of the first line 41 in parallel to one another and the units 106 through 110 of the second unit group are provided on the downstream side fluid line 41B of the first line 41 in parallel to one another. In addition, the units 111 through 115 of the third unit group are provided on the downstream side fluid line 42B of the second line 42 in parallel to one another and the units 116 through 120 of the fourth unit group are provided on the upstream side fluid line 42A of the second line 42 in parallel to one another.

As illustrated in Fig. 5, on the upstream side fluid line 41A of the first line 41, the first heat exchanger 71 is provided between the first pump 61 and the first unit group including the units 101 through 105. In addition, on the downstream side fluid line 41B of the first line 41, the second heat exchanger 72 is provided between the second pump 62 and the second unit group including the units 106 through 110. In addition, on the upstream side fluid line 42A of the second line 42, a third heat exchanger 73 is provided between the third pump 63 and the fourth unit group including the units 116 through 120. In addition, on the downstream side fluid line 42B of the second line 42, a fourth heat exchanger 74 is provided between the fourth pump 64 and the third unit group including the units 111 through 115.

As illustrated in Fig. 5, the dehydrator 1 includes a third tank 4A and the fourth tank 4B, into which the water separated from the treated fluid flows. As illustrated in Fig. 5, the first unit group including the units 101 through 105 and the third unit group including the units 111 through 115 are connected to the third tank 4A via the steam valve. Accordingly, the steam drawn out by the first unit group including the units 101 through 105 and the third unit group including the units 111 through 115 flows into the third tank 4A. In addition, the second unit group including the units 106 through 110 and the fourth unit group including the units 116 through 120 are connected to the fourth tank 4B via the steam valve. Accordingly, the steam drawn out by the second unit group including the units 106 through 110 and the fourth unit group including the units 116 through 120 flows into the fourth tank 4B.

In the present exemplary embodiment, as illustrated in Fig. 5, the second line 42 includes a bypass line 10. The bypass line 10 is connected to the upstream side fluid line 42A of the second line 42 via the branch valve (not illustrated). As illustrated in Fig. 5, the bypass line 10 connects between the upstream side fluid line 42A of the second line 42 and the first tank 2. The bypass line 10 bypasses two unit groups, i.e., the third unit group including the units 111 through 115 and the fourth unit group including the units 116 through 120, and is configured to allow the treated fluid from the second tank 3 to flow directly into the first tank 2. Note that in the present exemplary embodiment, because one intermediate tank is provided, the bypass line 10 is provided so as to bypass two unit groups provided on the second line 42. On the other hand, if a plurality of intermediate tanks is provided, the bypass line can be connected so as to bypass one or more unit group provided on the second line 42 to be connected to the intermediate tanks.

Note although not illustrated in Fig. 5, similarly to the first exemplary embodiment described above, the dehydrator 1 includes a control unit configured to control the operation of the entire dehydrator 1. The control unit is configured to control the first through the fourth pumps 61 through 64, first open-close valves 201 through 220, and second open-close valves 301 through 320. In addition, although not illustrated in the drawing, each of the water separation membrane units 101 through 120 includes a detector for detecting the malfunction occurring on the membrane container. The detector is connected to the control unit. When the control unit receives the detection signal which indicates that malfunction has occurred to the membrane container, the control unit executes control for closing both the first open-close valves 201 through 220 and the second open-close valves 301 through 320 corresponding to the water separation membrane unit to which the malfunction has occurred, among the water separation membrane units 101 through 120. With the above-described configuration, the maintenance and the replacement can be carried out only for the water separation membrane unit to which the malfunction has occurred, among the water separation membrane units 101 through 120.

Now, an operation of the dehydrator 1 according to the present exemplary embodiment will be described below with reference to the attached drawings.

At the start of the operation, the treated fluid (a crude ethanol aqueous solution) is supplied to the first tank 2. The treated fluid is continuously supplied. As illustrated in Fig. 5, then, the first pump 61 pumps the treated fluid from the first tank 2 into the intermediate tank 9. The treated fluid that flows through the upstream side fluid line 41A of the first line 41 is heated by the first heat exchanger 71 and flows into the units 101 through 105 of the first unit group provided in parallel to one another. The steam drawn out by the first unit group including the units 101 through 105 during the operation described above flows into the third tank 4A. Then, the treated fluid that has passed through the first unit group including the units 101 through 105 flows into the intermediate tank 9.

Then, the second pump 62 pumps the treated fluid from the intermediate tank 9 into the second tank 3. The treated fluid that flows through the downstream side fluid line 41B of the first line 41 is heated by the second heat exchanger 72 and flows into the units 106 through 110 of the second unit group provided in parallel to one another. The steam drawn out by the second unit group including the units 106 through 110 during the operation described above flows into the fourth tank 4B. Then, the treated fluid that has passed through the second unit group including the units 106 through 110 flows into the second tank 3.

Then, the control unit receives the concentration measured by a second ethanol concentration meter (not illustrated) of the second tank 3. Then, if the concentration measured by the second ethanol concentration meter has not reached a predetermined concentration, then the control unit controls the third pump 63. In this control, the control unit determines how many times the treated fluid is to pass through each unit group until the concentration of the treated fluid reaches the predetermined concentration according to the current concentration, for example. If it is determined that two unit groups (the third and the fourth unit groups including the units 111 through 120) can be bypassed according to the current concentration, then the control unit controls the branch valve provided between the bypass line 10 and the upstream side fluid line 42A of the second line 42 so that the treated fluid flows through the bypass line 10. Otherwise, the control unit executes control so that the treated fluid flows through the upstream side fluid line 42A of the second line 42. The following operation is executed when the treated fluid flows through the upstream side fluid line 42A of the second line 42. The treated fluid that flows through the upstream side fluid line 42A of the second line 42 is heated by the third heat exchanger 73 and flows into the units 116 through 120 of the fourth unit group provided in parallel to one another. The steam drawn out by the fourth unit group including the units 116 through 120 during the operation described above flows into the fourth tank 4B. Then, the treated fluid that has passed through the fourth unit group including the units 116 through 120 flows into the intermediate tank 9.

Then, the fourth pump 64 pumps the treated fluid from the intermediate tank 9 to the first tank 2. The treated fluid that flows through the downstream side fluid line 42B of the second line 42 is heated by the fourth heat exchanger 74 and flows into the units 111 through 115 of the third unit group provided in parallel to one another. The steam drawn out by the third unit group including the units 111 through 115 during the operation described above flows into the third tank 4A. Then, the treated fluid that has passed through the third unit group including the units 111 through 115 flows into the first tank 2. The above-described operation is repeated until the concentration of the treated fluid reaches the predetermined concentration. Then, the treated fluid whose concentration has reached the predetermined concentration is finally recovered from the second tank 3 as product ethanol.

According to the dehydrator 1 of the present exemplary embodiment, the plurality of water separation membrane units 101 through 120 is divided into the first unit group including the units 101 through 105, the second unit group including the units 106 through 110, the third unit group including the units 111 through 115, and the fourth unit group including the units 116 through 120, and the units 101 through 105 of the first unit group are provided on the upstream side fluid line 41A of the first line 41 in parallel to one another, the units 106 through 110 of the second unit group are provided on the downstream side fluid line 41B of the first line 41 in parallel to one another, the units 111 through 115 of the third unit group are provided on the downstream side fluid line 42B of the second line 42 in parallel to one another, and the units 116 through 120 of the fourth unit group are provided on the upstream side fluid line 42A of the second line 42 in parallel to one another. Accordingly, if any malfunction has occurred to any of the water separation membrane units 101 through 120, the maintenance and the replacement can be carried out only for the water separation membrane unit to which the malfunction has occurred, among the water separation membrane units 101 through 120, without stopping the entire dehydrator 1. Accordingly, it is enabled to stably operate the dehydrator 1.

In addition, according to the dehydrator 1 of the present exemplary embodiment, the second line 42 includes the bypass line 10 and the bypass line 10 is configured to bypass two unit groups, i.e., the third unit group including the units 111 through 115 and the fourth unit group including the units 116 through 120, to allow the treated fluid to flow from the second tank 3 directly to the first tank 2. Accordingly, the number of times the treated fluid is to pass through the membrane container units can be appropriately changed. As a result, the concentration of the treated fluid can be finely adjusted.

In addition, according to the dehydrator 1 of the present exemplary embodiment, the plurality of water separation membrane units 101 through 120 is divided into four unit groups. Accordingly, the decrease of the velocity of flow of the treated fluid can be suppressed. As a result, the decrease of the performance of water separation by the membrane container can be suppressed. Furthermore, according to the dehydrator 1 of the present exemplary embodiment, the dehydrator 1 includes the intermediate tank 9, which is provided between the first tank 2 and the second tank 3. With the above-described configuration, because the treated fluid can be stored also in the intermediate tank 9, the entire dehydrator 1 can be operated while continuously supplying the treated fluid to the first tank 2. According to the dehydrator 1 of the present exemplary embodiment, the product ethanol can be continuously recovered by continuously supplying the ethanol aqueous solution, which is the material of the product ethanol, to the dehydrator 1.

### Fifth Exemplary Embodiment

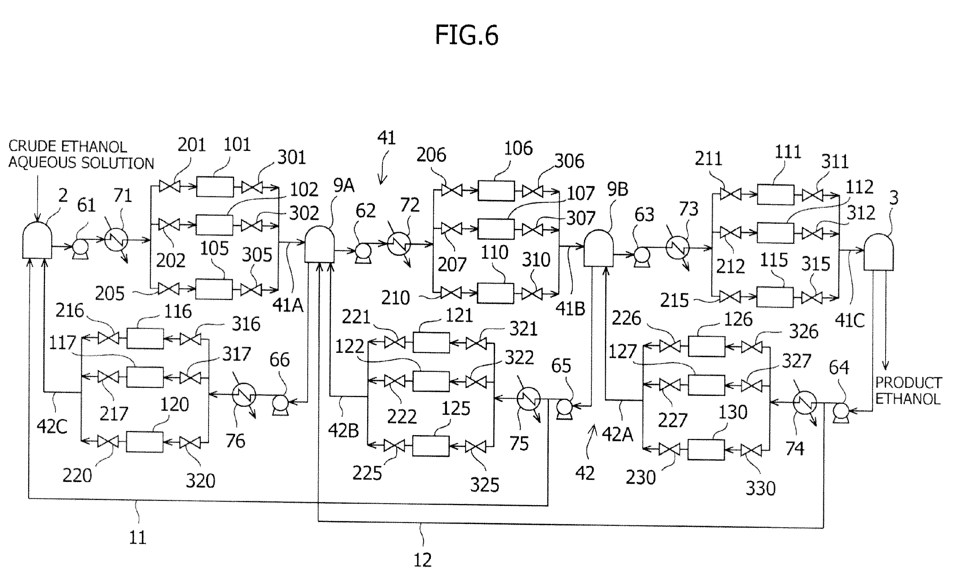

Now, the dehydrator 1 according to a fifth exemplary embodiment of the present invention will be described below with reference to the attached drawings. Fig. 6 is a schematic diagram illustrating the entire dehydrator 1 according to the fifth exemplary embodiment of the present invention. Note that the components similar to those of the above-described exemplary embodiments are provided with the same reference signs. Accordingly, the detailed description thereof will not be repeated here.

In the present exemplary embodiment, as illustrated in Fig. 6, the dehydrator 1 includes a first intermediate tank 9A and a second intermediate tank 9B, which are provided between the first tank 2 and the second tank 3. The first intermediate tank 9A and the second intermediate tank 9B are connected to both the first line 41 for allowing the treated fluid to flow from the first tank 2 to the second tank 3 and the second line 42 for allowing the treated fluid to flow from the second tank 3 to the first tank 2. In addition, the first intermediate tank 9A and the second intermediate tank 9B is provided with an ethanol concentration meter (not illustrated) for measuring the concentration of ethanol contained in the treated fluid.

Referring to Fig. 6, the first line 41 is constituted by the upstream side fluid line 41A for allowing the treated fluid to flow from the first tank 2 to the first intermediate tank 9A, an intermediate fluid line 41B for allowing the treated fluid to flow from the first intermediate tank 9A to the second intermediate tank 9B, and a downstream side fluid line 41C for allowing the treated fluid to flow from the second intermediate tank 9B to the second tank 3.

In addition, the second line 42 is constituted by the upstream side fluid line 42A for allowing the treated fluid to flow from the second tank 3 to the second intermediate tank 9B, an intermediate fluid line 42B for allowing the treated fluid to flow from the second intermediate tank 9B to the first intermediate tank 9A, and a downstream side fluid line 42C for allowing the treated fluid to flow from the first intermediate tank 9A to the first tank 2. Note that although Fig. 6 illustrates the embodiment in which two intermediate tanks (the first intermediate tank 9A and the second intermediate tank 9B) are provided, three or more intermediate tanks can be provided. If three or more intermediate tanks are provided, water separation membrane units can be provided among the intermediate tanks in parallel to one another.

Referring to Fig. 6, the upstream side fluid line 41A of the first line 41 is provided with the first pump 61 provided at a location close to the first tank 2. The intermediate fluid line 41B of the first line 41 is provided with the second pump 62, which is provided at a location close to the first intermediate tank 9A. The downstream side fluid line 41C of the first line 41 is provided with the second pump 63, which is provided at a location close to the second intermediate tank 9B.

In addition, the upstream side fluid line 42A of the second line 42 is provided with the fourth pump 64, which is provided at a location close to the second tank 3. The intermediate fluid line 42B of the second line 42 is provided with a fifth pump 65, which is provided at a location close to the second intermediate tank 9B. The downstream side fluid line 42C of the second line 42 is provided with a sixth pump 66, which is provided at a location close to the first intermediate tank 9A.

In the present exemplary embodiment, as illustrated in Fig. 6, the dehydrator 1 includes thirty water separation membrane units through 101 through 130. The plurality of water separation membrane units 101 through 130 is divided into the first unit group including the units 101 through 105, the second unit group including the units 106 through 110, the third unit group including the units 111 through 115, the fourth unit group including the units 116 through 120, a fifth unit group including the units 121 through 125, and a sixth unit group including the units 126 through 130.

As illustrated in Fig. 6, the units 101 through 105 of the first unit group are provided on the upstream side fluid line 41A of the first line 41 in parallel to one another, the units 106 through 110 of the second unit group are provided on the intermediate fluid line 41B of the first line 41 in parallel to one another, and the units 111 through 115 of the third unit group are provided on the downstream side fluid line 41C of the first line 41 in parallel to one another. In addition, the units 116 through 120 of the fourth unit group are provided on the downstream side fluid line 42C of the second line 42 in parallel to one another, the units 121 through 125 of the fifth unit group are provided on the intermediate fluid line 42B of the second line 42 in parallel to one another, and the units 126 through 130 of the sixth unit group are provided on the upstream side fluid line 42A of the second line 42 in parallel to one another.

As illustrated in Fig. 6, on the upstream side fluid line 41A of the first line 41, the first heat exchanger 71 is provided between the first pump 61 and the first unit group including the units 101 through 105. In addition, on the intermediate fluid line 41B of the first line 41, the second heat exchanger 72 is provided between the second pump 62 and the second unit group including the units 106 through 110. Furthermore, on the downstream side fluid line 41C of the first line 41, the third heat exchanger 73 is provided between the third pump 63 and the third unit group including the units 111 through 115. Moreover, on the upstream side fluid line 42A of the second line 42, the fourth heat exchanger 74 is provided between the third pump 63 and the sixth unit group including the units 126 through 130. In addition, on the intermediate fluid line 42B of the second line 42, a fifth heat exchanger 75 is provided between the fifth pump 65 and the fifth unit group including the units 121 through 125. Moreover, on the downstream side fluid line 42C of the second line 42, a sixth heat exchanger 76 is provided between the sixth pump 66 and the fourth unit group including the units 116 through 120.

In the present exemplary embodiment, as illustrated in Fig. 6, the second line 42 includes a first bypass line 11 and a second bypass line 12. The first bypass line 11 is connected to the intermediate fluid line 42B of the second line 42 via a branch valve (not illustrated). As illustrated in Fig. 6, the first bypass line 11 connects between the intermediate fluid line 42B of the second line 42 and the first tank 2. The first bypass line 11 is configured to bypass two unit groups, i.e., the fourth unit group including the units 116 through 120 and the fifth unit group including the units 121 through 125, to allow the treated fluid to flow directly from the second intermediate tank 9B into the first tank 2. The second bypass line 12 is connected to the upstream side fluid line 42A of the second line 42 via a branch valve (not illustrated). As illustrated in Fig. 6, the second bypass line 12 connects between the upstream side fluid line 42A of the second line 42 and the first intermediate tank 9A. The second bypass line 12 is configured to bypass two unit groups, i.e., the fifth unit group including the units 121 through 125 and the sixth unit group including the units 126 through 130, to allow the treated fluid to flow directly from the second tank 3 into the first intermediate tank 9A. Note that in the present exemplary embodiment, the first and the second bypass lines 11 and 12 bypass two unit groups provided on the second line 42. However, if a plurality of intermediate tanks is provided, the bypass lines can be provided so as to bypass one or more unit groups provided on the second line 42 to be connected to the intermediate tanks.

In addition, although not illustrated in Fig. 6, the dehydrator 1 includes a plurality of tanks into which the water separated from the treated fluid flows, similarly to the above-described fourth exemplary embodiment. The respective water separation membrane units 101 through 130 are connected to the plurality of tanks. Accordingly, the steam drawn out by each of the water separation membrane units 101 through 130 flows into the tanks.

In addition, although not illustrated in Fig. 6, similarly to the first exemplary embodiment described above, the dehydrator 1 includes a control unit configured to control the operation of the entire dehydrator 1. The control unit is configured to control the first through the sixth pumps 61 through 66, first open-close valves 201 through 230, and second open-close valves 301 through 330. In addition, although not illustrated in the drawing, each of the water separation membrane units 101 through 130 includes a detector for detecting the malfunction occurring on the membrane container. The detector is connected to the control unit. When the control unit receives the detection signal which indicates that malfunction has occurred to the membrane container, the control unit executes control for closing both the first open-close valves 201 through 230 and the second open-close valves 301 through 330 corresponding to the water separation membrane unit to which the malfunction has occurred, among the water separation membrane units 101 through 130. With the above-described configuration, the maintenance and the replacement can be carried out only for the water separation membrane unit to which the malfunction has occurred, among the water separation membrane units 101 through 130.

Now, an operation of the dehydrator 1 according to the present exemplary embodiment will be described below with reference to the attached drawings.

At the start of the operation, the treated fluid (a crude ethanol aqueous solution) is supplied to the first tank 2. The treated fluid is continuously supplied. As illustrated in Fig. 6, then, the first pump 61 pumps the treated fluid from the first tank 2 into the first intermediate tank 9A. The treated fluid that flows through the upstream side fluid line 41A of the first line 41 is heated by the first heat exchanger 71 and flows into the units 101 through 105 of the first unit group, which are provided in parallel to one another. Then, the treated fluid that has passed through the first unit group including the units 101 through 105 flows into the first intermediate tank 9A.

Then, the second pump 62 pumps the treated fluid from the first intermediate tank 9A into the second intermediate tank 9B. The treated fluid that flows through the downstream side fluid line 41B of the first line 41 is heated by the second heat exchanger 72 and flows into the units 106 through 110 of the second unit group provided in parallel to one another. Then, the treated fluid that has passed through the second unit group including the units 106 through 110 flows into the second intermediate tank 9B.

Then, the third pump 63 pumps the treated fluid from the second intermediate tank 9B to the second tank 3. The treated fluid that flows through the downstream side fluid line 41C of the first line 41 is heated by the third heat exchanger 73 and flows into the units 111 through 115 of the third unit group provided in parallel to one another. Then, the treated fluid that has passed through the third unit group including the units 111 through 115 flows into the second tank 3.

Then, the control unit receives the concentration measured by a second ethanol concentration meter (not illustrated) of the second tank 3. Then, if the concentration measured by the second ethanol concentration meter has not reached a predetermined concentration, then the control unit controls the fourth pump 64. In this control, the control unit determines how many times the treated fluid is to pass through each unit group until the concentration of the treated fluid reaches the predetermined concentration according to the current concentration, for example. If it is determined that two unit groups (the fifth and the sixth unit groups including the units 121 through 130) can be bypassed according to the current concentration, then the control unit controls the branch valve provided between the second bypass line 12 and the upstream side fluid line 42A of the second line 42 so that the treated fluid flows through the second bypass line 12. Otherwise, the control unit executes control so that the treated fluid flows through the upstream side fluid line 42A of the second line 42. The following operation is executed when the treated fluid flows through the upstream side fluid line 42A of the second line 42. The treated fluid that flows through the upstream side fluid line 42A of the second line 42 is heated by the fourth heat exchanger 74. The treated fluid then flows into the units of the sixth unit group including the units 126 through 130 provided in parallel to one another. Then, the treated fluid that has passed through the sixth unit group including the units 126 through 130 flows into the second intermediate tank 9B.

Then, the control unit receives the concentration measured by the second ethanol concentration meter of the second intermediate tank 9B. Consequently, the control unit controls the fifth pump 65. In this control, the control unit determines how many times the treated fluid is to pass through each unit group until the concentration of the treated fluid reaches the predetermined concentration according to the current concentration. If it is determined that two unit groups (the fourth and the fifth unit group including the units 116 through 125) can be bypassed according to the current concentration, then the control unit controls the branch valve provided between the first bypass line 11 and the intermediate fluid line 42B of the second line 42 so that the treated fluid flows through the first bypass line 11. Otherwise, the control unit executes control so that the treated fluid flows through the intermediate fluid line 42B of the second line 42. The following operation is executed when the treated fluid flows through the intermediate fluid line 42B of the second line 42. The treated fluid that flows through the intermediate fluid line 42B of the second line 42 is heated by the fifth heat exchanger 75. The treated fluid then flows into the units of the fifth unit group including the units 121 through 125 provided in parallel to one another. Then, the treated fluid that has passed through the fifth unit group including the units 121 through 125 flows into the first intermediate tank 9A.

Then, the sixth pump 66 pumps the treated fluid from the first intermediate tank 9A into the first tank 2. The treated fluid that flows through the downstream side fluid line 42C of the second line 42 is heated by the sixth heat exchanger 76. The treated fluid then flows into the units of the fourth unit group including the units 116 through 120 provided in parallel to one another. Then, the treated fluid that has passed through the fourth unit group including the units 116 through 120 flows into the first tank 2. The above-described operation is repeated until the concentration of the treated fluid reaches the predetermined concentration. Then, the treated fluid whose concentration has reached the predetermined concentration is finally recovered from the second tank 3 as product ethanol.

According to the dehydrator 1 of the present exemplary embodiment, the plurality of water separation membrane units 101 through 130 is divided into the first unit group including the units 101 through 105, the second unit group including the units 106 through 110, the third unit group including the units 111 through 115, the fourth unit group including the units 116 through 120, the fifth unit group 121 through 125, and the sixth unit group including the units 126 through 130. Furthermore, the units 101 through 105 of the first unit group are provided on the upstream side fluid line 41A of the first line 41 in parallel to one another, the units 106 through 110 of the second unit group are provided on the intermediate fluid line 41B of the first line 41 in parallel to one another, the units 111 through 115 of the third unit group are provided on the downstream side fluid line 41C of the first line 41 in parallel to one another, the units 116 through 120 of the fourth unit group are provided on the downstream side fluid line 42C of the second line 42 in parallel to one another, the units 121 through 125 of the fifth unit group are provided on the intermediate fluid line 42B of the second line 42 in parallel to one another, and the units 126 through 130 of the sixth unit group are provided on the upstream side fluid line 42A of the second line 42 in parallel to one another. Accordingly, if any malfunction has occurred to any of the water separation membrane units 101 through 130, the maintenance and the replacement can be carried out only for the water separation membrane unit to which the malfunction has occurred, among the water separation membrane units 101 through 130, without stopping the entire dehydrator 1. Accordingly, it is enabled to stably operate the dehydrator 1.

In addition, according to the dehydrator 1 of the present exemplary embodiment, the second line 42 includes the first bypass line 11 and the second bypass line 12. The first bypass line 11 is configured to bypass two units, i.e., the fourth unit group including the units 116 through 120 and the fifth unit group including the units 121 through 125, to allow the treated fluid to flow directly from the second intermediate tank 9B into the first tank 2, and the second bypass line 12 is configured to bypass the fifth unit group including the units 121 through 125 and the sixth unit group including the units 126 through 130 to allow the treated fluid to flow directly from the second tank 3 into the first intermediate tank 9A. Accordingly, the number of times the treated fluid is to pass through the membrane container units can be appropriately changed. As a result, the concentration of the treated fluid can be finely adjusted.

In addition, according to the dehydrator 1 of the present exemplary embodiment, the plurality of water separation membrane units 101 through 130 is divided into six unit groups. Accordingly, the decrease of the velocity of flow of the treated fluid can be suppressed. As a result, the decrease of the performance of water separation by the membrane container can be suppressed. Moreover, according to the dehydrator 1 of the present exemplary embodiment, the dehydrator 1 is provided with the first intermediate tank 9A and the second intermediate tank 9B, which are provided between the first tank 2 and the second tank 3. With the above-described configuration, because the treated fluid can be stored also in the first intermediate tank 9A and the second intermediate tank 9B, the entire dehydrator 1 can be operated while continuously supplying the treated fluid to the first tank 2. According to the dehydrator 1 of the present exemplary embodiment, the product ethanol can be continuously recovered by continuously supplying the ethanol aqueous solution, which is the material of the product ethanol, to the dehydrator 1.

Exemplary embodiments of the present invention are described above. However, the present invention is not limited to the exemplary embodiments described above and can be implemented by various modifications and alterations based on the technical idea of the present invention.

In the first through the third exemplary embodiments, ten water separation membrane units are used. In the fourth exemplary embodiment, twenty water separation membrane units are used. In the fifth exemplary embodiment, thirty water separation membrane units are used. However, the number of the water separation membrane units of the present invention is not limited to these numbers. To paraphrase this, the number of the water separation membrane units can be appropriately changed according to the flow rate of the treated fluid.

In the fourth and the fifth exemplary embodiments, the heat exchanger is provided for each fluid line. However, if the change in the temperature of the treated fluid is small, it is not necessary to provide the heat exchanger for all the fluid lines.

In the fifth exemplary embodiment, the first bypass line 11 and the second bypass line 12 are configured to bypass two unit groups including the water separation membrane units. However, the bypass lines of the present invention are not limited to those described above. To paraphrase this, it is useful if bypass lines configured to bypass three unit groups to allow the treated fluid to flow directly from the second tank 3 into the first tank 2 is provided.

**Reference Signs List**

| | |
|---|---|
| 1: | Dehydrator |
| 2: | First tank |
| 3: | Second tank |
| 2a, 3a: | Ethanol concentration meter |
| 4: | Third tank |
| 5: | Control unit |
| 9, 9A, 9B: | Intermediate tank |
| 10, 11, 12: | Bypass line |
| 41: | First line |
| 42: | Second line |
| 41A, 41B, 41C: | Fluid line for the first line (first fluid line) |
| 42A, 42B, 42C: | Fluid line for the second line (second fluid line) |
| 61, 62, 63, 64, 65, 66: | Pump |
| 71, 72, 73, 74, 75, 76: | Heat exchanger |
| 81, 82: | Bypass tube |
| 101 through 130: | Water separation membrane unit (membrane container unit) |
| | 101a through 110a Detector |
| 201 through 230: | First open-close valve |
| 301 through 330: | Second open-close valve |

## Claims

1. A dehydrator configured to separate water from treated fluid comprising:
a first tank configured to store the treated fluid before water is separated therefrom;
a second tank into which the treated fluid whose water has been separated therefrom flows; and
a plurality of membrane container units having a separation membrane configured to separate water from the treated fluid, provided between the first tank and the second tank and in parallel to one another along a direction of flow of the treated fluid,
wherein the treated fluid is configured to reciprocate between the first tank and the second tank, and
wherein the treated fluid is configured to pass through the plurality of membrane container units for a plurality of times.

2. The dehydrator according to claim 1, further comprising:
a first line configured to allow the treated fluid to reciprocate between the first tank and the second tank,
wherein the plurality of membrane container units is arranged on the first line in parallel to one another,
wherein the treated fluid is configured, after all the treated fluid has flowed from the first tank to the second tank via the plurality of membrane container units provided on the first line and no treated fluid remains in the first tank, to flow from the second tank to the first tank via the plurality of membrane container units provided on the first line.

3. The dehydrator according to claim 1, further comprising:
a first line configured to allow the treated fluid to flow from the first tank to the second tank; and
a second line configured to allow the treated fluid to flow from the second tank to the first tank,
wherein the plurality of membrane container units is arranged on the first line in parallel to one another,
wherein the treated fluid is configured, after all the treated fluid has flowed from the first tank to the second tank via the plurality of membrane container units provided on the first line and no treated fluid remains in the first tank, to flow from the second tank to the first tank via the second line.

4. The dehydrator according to claim 1, further comprising:
a first line configured to allow the treated fluid to flow from the first tank to the second tank; and
a second line configured to allow the treated fluid to flow from the second tank to the first tank,
wherein the plurality of membrane container units is divided into a first unit group including units provided on the first line and in parallel to one another and a second unit group including units provided on the second line and in parallel to one another, and
wherein the treated fluid is configured, after all the treated fluid has flowed from the first tank to the second tank via the first unit group provided on the first line and no treated fluid remains in the first tank, to flow from the second tank to the first tank via the second unit group provided on the second line.

5. The dehydrator according to claim 1, further comprising:
a first line configured to allow the treated fluid to flow from the first tank to the second tank;
a second line configured to allow the treated fluid to flow from the second tank to the first tank; and
at least one intermediate tank provided between the first tank and the second tank and connected to both the first line and the second line,
wherein the first line is divided into a plurality of first fluid lines provided across the intermediate tank and the second line is divided into a plurality of second fluid lines provided across the intermediate tank,
wherein the plurality of membrane container units is divided into unit groups including as many units as a total number of the first fluid line and the second fluid line and units included in the unit group of the plurality of membrane container units are provided in parallel to one another on each of the first fluid line and the second fluid line,
wherein the treated fluid is configured to flow from the first tank to the second tank successively via the units included in the unit group provided on the first line and the intermediate tank and is configured to flow from the second tank to the first tank successively via the units included in the unit group provided on the second line and the intermediate tank,
wherein the second line includes at least one bypass line, and
wherein the bypass line is configured to bypass one or more units of the unit group provided on the second line, to allow the treated fluid to return to the intermediate tank or the first tank.
